(19) Europäisches Patentamt
European Patent Office
Office européen des brevets

(11) **EP 2 615 461 A1**

(12) **EUROPEAN PATENT APPLICATION**

(43) Date of publication:
**17.07.2013 Bulletin 2013/29**

(51) Int Cl.:
***G01N 33/74*** *(2006.01)*

(21) Application number: **12305056.9**

(22) Date of filing: **16.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Cisbio Bioassays**
**30200 Codolet (FR)**
• **INSTITUT NATIONAL DE LA SANTE ET DE LA RECHERCHE MEDICALE (INSERM)**
**75654 Paris Cédex 13 (FR)**
• **Université de Strasbourg**
**67000 Strasbourg (FR)**

(72) Inventors:
• **Garnero, Patrick**
**30330 Gaujac (FR)**

• **Metz-Boutigue, Marie-Hélène**
**67000 Strasbourg (FR)**
• **Schneider, Francis**
**67000 Strasbourg (FR)**
• **Lavigne, Thierry**
**67000 Strasbourg (FR)**
• **Corti, Angelo**
**24052 Azzano San Paolo**
**Bergamo (IT)**
• **Crippa, Luca**
**20871 Vimercate (IT)**

(74) Representative: **Nevant, Marc et al**
**Cabinet Beau de Loménie**
**158, rue de l'Université**
**75340 Paris Cedex 07 (FR)**

(54) **Method for predicting a risk of increased mortality**

(57) The invention relates to a method for determining if a patient is at risk of increased mortality, said method comprising the step of measuring the level of vasostatin-1 in a biological sample obtained from said patient. The invention also relates to a sandwich immunoassay kit for implementing said method.

FIG.1

**Description**

**FIELD OF THE INVENTION**

The present invention relates to a method for predicting a risk of increased mortality, particularly in critically ill patients admitted to emergency services or Intensive Care Units.

**BACKGROUND OF THE INVENTION**

Critically ill patients are usually admitted with clinical and biochemical signs of systemic inflammation as a consequence of their diseases. These signs are ascribed to the effects of inflammatory mediators produced during the early phase response to a stress of unspecified etiology. The systemic inflammatory response syndrome (SIRS) is the clinical response traditionally associated with this biological inflammation: its incidence can be more than 50% in critically ill patients, many of whom demonstrate or will develop severe infection (Brun-Buisson. Intensive Care Med. 2000; 26 Suppl 1:S64-74).

C-reactive protein (CRP) and procalcitonin are the traditional biomarkers used to evaluate the level of biological inflammation and infection, respectively. Despite their sensitivity and specificity for the diagnosis of infection, neither procalcitonin nor CRP give significant information as to outcome in such patients (Harbarth et al. Am J Respir Crit Care Med.2001;164:396-402), who often subsequently die with multiple organ failure (MOF). Most patients surviving initial hemodynamic failure experience an insidious, progressive decline of vital organ functions, corresponding to multiple organ failure, which is associated with high and short-term mortality rates. Mortality varies from 30% to 100% even in the absence of previous health disorders.

Attempts to characterise the severity of organ failures and to predict patient outcome is of major importance for physicians in the care of critically ill patients. From an historical point of view, score building was the first step to evaluate outcome in critically ill patients because previous attempts with biological parameters failed to succeed (Schetz et al. Int J Artif Organs 2005;28(2):1197-210). Several outcome prediction models have been developed and are currently available in intensive care unit management, such as APACHE (Acute Physiology and Chronic Health Evaluation) (Knaus et al. Crit Care Med. 1981 ;9:591 - 597), SAPS (simplified acute physiology score) (Le Gall et al.. JAMA 1993; 270: 2957- 2963), MPM (mortality probability models) (Lemeshow et al. Crit Care Med. 1988; 16:470- 477), SOFA (Sequential Organ Failure Assessment (SOFA) (Ferreira et al. JAMA 2001; 286: 1754-1758), and LOD (Logistic Organ Dysfunction) (Timsit et al. Crit Care Med 2002; 30: 2003-1 3). Among these approaches, SAPS is considered as the gold standard score.

However, these prediction models have several drawbacks. First of all, a calculation of score is generally only available 24 h after Intensive Care Unit (ICU) admission. Generally, more than 10 parameters shall be evaluated for determining the score. Furthermore, these scores ignore the many factors that can influence patient outcome during the course of an ICU stay. Finally, it has been shown that these scoring systems are more useful to describe and quantify organ function than to predict a real outcome.

International patent application WO2008/145701 discloses a method for predicting the outcome of a critically ill patient, based on the assay of chromogranin A (CgA) or a fragment thereof in a biological sample obtained from a patient. The results presented in this document showed that CgA could be used as an early biomarker of SIRS, and an efficient tool to predict the outcome of a critically ill patient. However, this study was conducted on a limited number of patients, was monocentric and patients had only medical (i.e.: non-surgical) causes of referral. Moreover, the CgA test that was used detects the median core portion of the CgA molecule, which is present in numerous fragments of CgA and therefore did not allow to precisely follow the generation of specific fragments.

CgA is known to be processed through its numerous dibasic sites by the prohormone convertases PC1/3 and PC2, co-stored in the granules with CgA. This intracellular and extracellular processing leads to several biologically CgA-derived peptides. Among them, vasostatin 1 (VS-1, CgA 1-76) has shown to have different biological activities. It inhibits vascular endothelial growth factor (VEGF) which suggests a potential inhibitory effect on tumor growth via depressing endothelial proliferation and migration. VS-1 has also antifungal activities via its chromofungin sequence, and it regulates the cardiovascular system

An immunoassay of VS-1 has been described by Roatta et al. (Regul Pept. 2011 Jun 7; 168(1-3): 10-20). This team measured serum concentrations of VS-1 using a sandwich ELISA based on the anti-CgA monoclonal antibody 5A8 (capture step) and the biotinylated anti-CgA mAb B4E11 (detection step). Those two antibodies had been previously described and recognize epitopes corresponding to amino-acids 53-57 (mAb 5A8) and 68-79 (mAb B4E11) of chromogranin A. This assay was therefore not specific of 1-76 VS-1 since it could also measure fragments including amino acids 78 and 79 which are not part of circulating 1-76 VS-1.

There is therefore a need for new biomarkers that could help determine if a patient, and particularly a critically ill patient, is at risk of increased short term mortality.

**DESCRIPTION**

The inventors have now discovered that Vasostatin-1 is a new biomarker that can be used to determine if a patient is at risk of increased mortality. 1-76 Vasostatin-1 (hereafter "*VS-1*") is the highly conserved N-terminal 1-76 fragment of chromogranin A (amino-acid 19-94 of chromogranin A as described in entry P10645 of Uniprot database) and is released after major endogenous cleavages in the secretory granules of nervous, endocrine and immune cells. Its initial property was considered to be its ability to relax precontracted vessels, but VS-1 was later shown to stabilize heart functioning by counterbalancing adrenergic-dependent stress.

The present invention relates to a method for determining if a patient is at risk of increased mortality, said method comprising the step of:

(a) measuring the level of VS-1 in a biological sample obtained from said patient.

Preferably this method further comprises the steps of:

(b) comparing the measured level of VS-1 in the sample with a predetermined value measured in a control population of healthy subjects or critically ill survivors; and
(c) correlating levels of VS-1 with the patient's mortality risk.

In a particular embodiment of this method, step (b) further comprises comparing the age of the patient with a predetermined cut-off age and step (c) consists in correlating age and level of VS-1 with the patient's mortality risk.

The lactate level is routinely measured to help determine if someone has lactic acidosis, a high level of lactate in the blood. Lactic acidosis is most commonly caused by an inadequate amount of oxygen in cells and tissues (hypoxia), which can result from an acute condition, such as sepsis, shock or heart attack, or a chronic condition, such as severe congestive heart failure. The inventors have now discovered that it is particularly advantageous to combine the measurement of VS-1 level with that of lactate in order to determine if a patient is at risk of increased mortality.

Consequently, in a preferred embodiment of the invention, both VS-1 and lactate levels are used. In this embodiment, the method for determining if a patient is at risk of increased mortality comprises the steps of:

(a) measuring the level of VS-1 in a biological sample obtained from said patient;
(b) measuring the level of lactate in a biological sample obtained from said patient.

In this embodiment, the biological sample used in step (b) can be the same sample as the one used in step (a).

Preferably, this embodiment of the invention further comprises the steps of:

(c) comparing the measured level of VS-1 and lactate in the samples with predetermined values of each marker measured in control populations of healthy subjects or critically ill survivors; and
(d) correlating levels of VS-1 and of lactate with the patient's mortality risk.

In another embodiment of the invention, which can be combined with previous or further embodiments, the age of the patient is taken into account to determine if a patient is at risk of increased mortality. In this case, step (c) of the previous embodiment further comprises comparing the age of the patient with a predetermined cut-off age and step (d) consists in correlating age and levels of VS-1 and lactate with the patient's mortality risk.

The method according to the invention can be used for monitoring the increase or decrease of a patient's mortality risk. Such method can be used to monitor the effectiveness and/or progress of therapy in a subject. Therefore, the invention also relates to a method of monitoring the decrease or increase of a patient mortality risk, comprising implementing the method described above with a first and a second or further sample from the same patient, said first and second or further samples being obtained at different times, correlating VS-1 level and optionally lactate level and/or optionally age with the patient's mortality risk for each of the first or second or further sample, and then comparing the patient's mortality risk determined with each sample.

In all embodiments of the invention, the step of measuring the level of VS-1 in a biological sample preferably comprises the steps of:

(i) contacting said biological sample with a compound capable of binding VS-1;
(ii) detecting the level of VS-1 present in the sample by observing the level of binding between said compound and VS-1.

The compound capable of binding VS-1 is preferably an antibody raised against vasostatin-1 or synthetic peptides derived therefrom. In one embodiment, the antibody's epitope is located within amino acids 66 to 76 of VS-1.

In a yet another embodiment of the invention, which can be combined with previous or further embodiments, the patient is a critically ill patient, notably a patient affected with severe SIRS, severe sepsis or septic shock.

Finally, in another aspect, the invention relates to a VS-1 sandwich immunoassay kit comprising a first antibody whose epitope is located within amino-acids 66 to 76 of VS-1, preferably within amino acids 70 to 76, and a second antibody whose epitope is located within amino-acids 1 to 66 of VS-1.

*Definitions:*

The term "*systemic inflammatory response syndrome (SIRS)*" describes a serious condition in which there is inflammation throughout the whole body. It may be caused by a severe bacterial infection (sepsis), trauma, pancreatitis drug reactions, autoimmune diseases, and other disorders. It is characterized by increased heart rate, low blood pressure, low or high body temperature, and low or high white blood cell count. Criteria for SIRS were established in 1992 as part of the American College of Chest Physicians/Society of Critical Care Medicine Consensus Conference. The conference concluded that the manifestations of SIRS include, but are not limited to:

- Body temperature less than 36°C or greater than 38°C
- Heart rate greater than 90 beats per minute
- Tachypnea (high respiratory rate), with greater than 20 breaths per minute; or an arterial partial pressure of carbon dioxide less than 4.3 kPa (32 mmHg)
- White blood cell count less than 4000 cells/mm$^3$ (4 x 10$^9$ cells/L) or greater than 12,000 cells/mm$^3$ (12 x 10$^9$ cells/L); or the presence of greater than 10% immature neutrophils (band forms).

SIRS can be diagnosed when two or more of these criteria are present.

The term "*severe SIRS*" describes a condition where SIRS is combined with organ dysfunction.

The terms "*sepsis*", "*severe sepsis*" and "*septic shock*" are used to identify the continuum of the clinical response to infection. Patients with sepsis present evidences of infection and clinical manifestations of SIRS. Patients with severe sepsis develop hypoperfusion with organ dysfunction. Septic shock is manifested by hypoperfusion and persistent hypotension.

The term "*organ dysfunction*" is defined as a clinical syndrome in which the development of progressive and potentially reversible physiological dysfunction in an organ is characteristic even in the absence of anatomic injuries.

The term "*multiple organ failure*" or "*MOF*" denotes an organ dysfunction occurring in 2 or more organs.

In the context of the present invention, the term "*level*" refers to a value reflecting the quantity of the molecular entity (such as VS-1 or lactate) mentioned in the respective context.

The terms "*biological sample*" as used herein refer to a biological sample obtained for the purpose of in vitro evaluation. In the methods of the present invention, the sample or patient sample preferably may comprise any body fluid. Typical biological samples to be used in the method according to the invention are blood samples (e.g. whole blood sample, serum sample, or plasma sample). Other possible biological samples include urine, saliva, bronchio alveolar fluid, synovial fluid, spinal fluid, stools and tissue sample.

Typically the biological sample may be obtained from a critically ill patient at the admission at a critical care unit or at an emergency ward.

By the terms "*at the admission*", it is meant within a couple of hours of the time the patient is considered for medical care at admission in a critical care unit or at an emergency ward. Biological samples can also be obtained within up to 72 hours after admission, since it has been discovered that VS-1 levels are stable in critically ill patients at least over that period of time.

The invention is based on the following findings:

(i) Critically ill patients exhibit significantly higher plasma VS-1 concentrations at their admission in ICU, when compared with healthy volunteers.

(ii) Only severe SIRS patients, severe sepsis and septic shock patients exhibit significantly higher plasma VS-1 concentrations at admission when compared to no SIRS / no infection patients and sepsis patients.

(iii) Plasma VS-1 concentration at admission is significantly higher in non survivors when compared to critically ill survivors, i.e. patients who survived past 28 days after admission in ICU.

(iv) Lactate plasmatic concentration and age are predictive markers of mortality; it has now been found that lactate and VS-1 concentrations are independent biomarkers of mortality. Surprisingly, when combining lactate, VS-1 levels and age, using the best threshold for each independent factor of mortality prediction, a predictive score has been calculated using a multivariate logistic regression model and this score appears to be significantly better than each factor taken alone.

Those findings support the fact that elevated VS-1 concentration in a patient sample is a reliable indicator of a risk of increased mortality (particularly, short term mortality, i.e. within 28 days after admission or 28 days after VS-1 level has been determined). Interestingly, the method according to the invention does not require specifying an initial diagnostic or category of patients (such as patient suffering from a medical condition, a trauma or having been subject to a surgical procedure), and can therefore be used very early on after admission of any kind of patients. It is however particularly appropriate for critically or acutely ill patients, in intensive care units.

The invention involves correlating levels of VS-1, optionally in combination with lactate levels or with lactate levels and age, with the mortality risk.

The term "correlating" means that the skilled artisan uses the marker(s) level(s) and optionally age to determine whether the patient is at risk of increased mortality, by comparing its/their value with a predetermined value(s) (a threshold value, or cut-off value) corresponding to the marker level in a reference population. This reference population can be a population of healthy subjects, or a population of critically ill survivors, i.e. critically ill patients who survived more than 28 days after admission. The term "healthy subject" is self explanatory for the skilled in the art.

The predetermined value can take a variety of forms. It can be a single cut-off value, such as for instance a median or mean or the $75^{th}$, $90^{th}$, $95^{th}$ or $99^{th}$ percentile of a population of healthy subjects or critically ill survivors. By using a higher percentile than the $75^{th}$ percentile, one reduces the number of false positive subjects identified, but one might miss to identify subjects, who are at moderate, albeit still increased risk. Thus, one might adopt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.

The predetermined value can vary among particular populations selected, depending on their habits, ethnicity, genetics etc. Accordingly, the predetermined values selected may take into account the category in which an individual falls. Appropriate ranges and categories can be selected with no more than routine experimentation by those of ordinary skill in the art.

In a preferred embodiment, the predetermined value for VS-1 level is the median of the VS-1 level in an ensemble of pre-determined samples in a population of healthy subjects or critically ill survivors.

In another preferred embodiment, this predetermined value is a quantile (preferably the $75^{th}$ percentile) of the level of VS-1 in an ensemble of pre-determined samples in a population of healthy subjects or critically ill survivors.

It has been found that the optimum cut-off value for VS-I plasmatic concentration to predict if a patient is at risk of increased mortality, is between 3 and 6 ng/mL, and most preferably between 4 and 5 ng/mL. Preferably, this cut-off value is around 4.51 ng/mL.

Similarly, the predetermined value for lactate level is the median or a quantile (preferably the $75^{th}$ percentile) of the lactate level in an ensemble of pre-determined samples in a population of healthy subjects or critically ill survivors.

It has been found that the optimum cut-off value for lactate plasmatic concentration to predict if a patient is at risk of increased mortality, is between 3 and 6 mmol/L, and most preferably between 4 and 5 mmol/L. Preferably, this cut-off value is around 4.56 mmol/L. As far as age goes, it has been determined that the optimum cut-off value is chosen from: 65, 66, 67, 68, 69, 70, 71, 72, 73, 74, 75, 76, 77, 78, 79, 80, 81, 82, 83, 84 years old. Preferably, this cut-off value is 77 years old.

The correlation of the level of a marker with a risk of increased mortality can consist in a simple comparison of the marker's level in the patient sample with a predetermined cut-off value, whereby when said marker level exceeds said cut-off value, the patient is determined to be at risk of increased mortality.

When more than one marker or parameter is used, such as when both VS-1 and lactate are used, their correlation with a risk of increased mortality can be based on the calculation of a score that combines the various markers, and which is then compared with a cut-off score. This score can be obtained from a formula based on Cox Proportional Hazards analysis or by using Risk index calculations such as the NRI (Net Reclassification Index) or the IDI (Integrated Discrimination Index). The indices can be calculated according to Pencina et al. (Evaluating the added predictive ability of a new marker: from area under the ROC curve to reclassification and beyond, Stat Med. 2008;27:157-172).

This embodiment is preferred when more than one parameter is used, i.e. in the embodiments where VS-1 is used in conjunction with lactate, with age or with lactate and age.

By using a multivariate logistic regression model, it has been determined that the following formula could be used to calculate the score:

$$\text{Score} = 1.0431 \text{ X } (0;1) \text{ age} + 1.114 \text{ X } (0;1) \text{ [lactate]} + 1.0324 \text{ X } (0;1) \text{ [VS-1]},$$

wherein "age" is the age of the patient in years, the lactate concentration is expressed in mmol/L, and the VS-1 concentration is expressed in ng/mL. When age, [lactate] or [VS-1] are greater than their associated individual cut-off value, then the value of those parameters is multiplied by the associated coefficient. When they are lower, their value is discarded from the score calculation.

It has also been determined that the optimal cut-off value for this score was 1.114 and that if a calculated score is greater than this value, then the patient should be considered as being at risk of increased mortality, with a sensitivity of 59% and a specificity of 85%. In that case, the positive likelihood ratio is greater than 3, and the negative likelihood ratio is lesser than 0.5.

For example if the age, lactate and VS-1 cut-off values are respectively 77 years old, 4.56 mmol/L and 4.51 ng/mL, and that a patient is 64 years old with 4.8 mmol/L of lactate and 4.2 ng/mL of VS-1, then:

Score = 1.0431 X 0 + 1.114 X 4.8 + 1.0324 X 0 = 5.34, which is above the cut-off value of 1.114 which means that this patient is at a risk of increased mortality.

The method and kit according to the invention are particularly useful to assess conditions of critically ill patients, i.e. patients whose lives are threatened and who may therefore die within a short period of time (hours or days), such as patients affected with severe SIRS, severe sepsis or severe shock. The invention is therefore extremely valuable in Intensive Care Units and emergency departments, where patients at risk of increased mortality may receive a more intensive treatment and attention than other patients. Such method and kit may thus help the physician to make a choice on a therapeutic treatment which can accordingly consist in administering accurate drugs to the patients, and is also useful to monitor the therapeutic effect of a drug. Costs of the treatments may therefore be adapted to the severity and morbidity of the patients admitted in intensive care units, and accordingly the method and kit of the invention may represent a useful tool for the management of such units.

## *VS-1 ASSAY*

Once the biological sample from the patient is prepared, the concentration of VS-1 may be measured by any known method in the art.

For example, the concentration of VS-1 may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; fluoroimmunoassays; luminescence based immunoassays; chemiluminescence based immunoassays; immunoelectrophoresis; immunoprecipitation; high performance liquid chromatography (HPLC); size exclusion chromatography; solid-phase affinity, etc.

In a particular embodiment, such methods comprise contacting the biological sample with a compound capable of selectively interacting with VS-1 present in the biological sample, and detecting the level of VS-1 present in the sample by observing the level of binding between said compound and VS-1.

In one embodiment, the compound capable of selectively interacting with VS-1 may be generally an antibody raised against VS-1 or synthetic peptides derived therefrom, that may be polyclonal or monoclonal, preferably monoclonal. In a preferred embodiment, this antibody binds to an epitope located within amino acids 66 to 76 of VS-1, preferably within amino acids 70 to 76 of VS-1.

Polyclonal antibodies directed against VS-1 can be raised according to known methods by administering the appropriate antigen or epitope to a host animal selected, e.g., from pigs, cows, horses, rabbits, goats, sheep, llama, and mice, among others. Various adjuvants known in the art can be used to enhance antibody production. Although antibodies useful in practicing the invention can be polyclonal, monoclonal antibodies are preferred. Monoclonal antibodies against VS-1 can be prepared and isolated using any technique that provides for the production of antibody molecules by continuous cell lines in culture. Techniques for production and isolation include but are not limited to the hybridoma technique originally described by Kohler et al. (Nature. 1975;256 (5517):495-7); the human B-cell hybridoma technique (Cote et al Proc Natl Acad Sci U S A. 1983;80(7):2026-30); and the EBV-hybridoma technique (Cole et al., 1985, In Monoclonal Antibodies and Cancer Therapy (Alan Liss, Inc.) pp. 77-96). Alternatively, techniques described for the production of single chain antibodies (see e.g. U.S. Pat. No. 4,946,778) can be adapted to produce anti-VS-1, single chain antibodies. Antibodies useful in practicing the present invention also include anti-VS-1 fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to VS-I. For example, phage display of antibodies may be used. In such a method, single-chain Fv (scFv) or Fab fragments are expressed on the surface of a suitable bacteriophage, e. g., M13. Briefly, circulating lymphocytes, spleen cells or lymph nodes of a suitable host, e. g., mouse, rabbit, llama that has been immunized with a protein, are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

In another embodiment, the compound capable of selectively interacting with VS-1 may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk et al. (Science 1990, 249, 505-510). The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena (Clin Chem. 1999; 45(9): 1628-50). Peptide aptamers consist of conformationally constrained antibody variable regions displayed by a platform protein, such as E. coli Thioredoxin A, that are selected from combinatorial libraries by two hybrid methods (Colas et al. Nature 1996, 380, 548-50).

The compounds capable of selectively interacting with VS-1, such as antibodies or aptamers, may be labeled with a detectable molecule or substance, such as a fluorescent molecule, a radioactive molecule, an enzyme such as horseradish peroxidase, or any others labels known in the art. Labels are known in the art that generally provide (either directly or indirectly) a signal.

As used herein, the term "labeled", with regard to the antibody, is intended to encompass direct labeling of the antibody or aptamer by coupling (i.e., physically linking) a detectable substance, such as a radioactive agent or a fluorophore (e.g. fluorescein isothiocyanate (FITC), phycoerythrin (PE), Indocyanine (Cy5), a FRET donor such as a fluorescent rare earth chelate or cryptate, or a FRET acceptor) to the antibody or aptamer, as well as indirect labeling of the probe or antibody by reactivity with a detectable substance. An antibody or aptamer of the invention may be labeled with a radioactive molecule by any method known in the art. For example radioactive molecules include but are not limited to radioactive atoms such as $I^{123}$, $I^{124}$, $I^{125}$, $In^{111}$, $Re^{186}$, $Re^{188}$.

The aforementioned assays generally involve the binding of the compound capable of selectively interacting with VS-1 (i.e. antibody or aptamer) in a solid support. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

More particularly, a sandwich method can be used, wherein the wells of a microtiter plate are coated with a capture antibody against VS-1. A biological sample containing or suspected of containing VS-1 is then added to the coated wells. After a period of incubation sufficient to allow the formation of antibody-antigen complexes, the plate(s) can be washed to remove unbound moieties and a detectably labeled secondary binding molecule added (the labeled antibody). The secondary binding molecule is allowed to react with any captured sample marker protein, the plate washed and the presence of the secondary binding molecule detected using methods well known in the art. In a preferred embodiment of the invention, VS-1 is measured using such a sandwich immunoassay, that includes a first antibody whose epitope is located within amino acids 66-76 of VS-1 (preferably within amino acids 70-76) and a second antibody whose epitope is located within amino acids 1-66 of VS-1 (preferably within amino acids 53-57).

The preferred assay formats are shown in table 1 with the sign "+", wherein lines correspond to the localization of the VS-1 epitope of the capture antibody and columns correspond to the localization of the VS-1 epitope of the labeled antibody:

**Table 1**

|          | Aa 66-76 | Aa-70-76 | Aa 1-66 | Aa 53-57 |
|----------|----------|----------|---------|----------|
| Aa 66-76 | -        | -        | +       | +        |
| Aa 70-76 | -        | -        | +       | +        |
| Aa 1-66  | +        | +        | -       | -        |
| Aa 53-57 | +        | +        | -       | -        |

Measuring the concentration of VS-1 (with or without immunoassay-based methods) may also include separation of the proteins: centrifugation based on the protein's molecular weight; electrophoresis based on mass and charge;

HPLC based on hydrophobicity; size exclusion chromatography based on size; and solid-phase affinity based on the protein's affinity for the particular solid-phase that is used. Once separated, VS-1 may be identified based on the known "separation profile" e.g., retention time, for that protein and measured using standard techniques. Alternatively, the separated proteins may be detected and measured by, for example, a mass spectrometer.

Homogeneous assays of VS-1 are particularly advantageous since they can be performed without washing or purification steps. In a preferred embodiment, VS-1 is measured with a Fluorescence Resonance Energy Transfer (FRET) assay, involving the use of energy donor and energy acceptor compounds coupled to e.g. antibodies of a sandwich immunoassay.

## *LACTATE ASSAY*

Lactate assay kits are commercially available and routinely performed in medical facilities.
Roche markets a device (Cobas) to measure lactate level.
Abcam and other companies provide a lactate assay fluoromoetric kit, where lactate is oxidized by lactate oxidase to generate a product, which interacts with a probe to produce colour and fluorescence. The kit provides a convenient means for detecting L(+)-Lactate in biological samples such as in blood circulation by either colorimetric or fluorometric methods.
The invention will further be illustrated in view of the following figures and examples.

## FIGURE LEGENDS

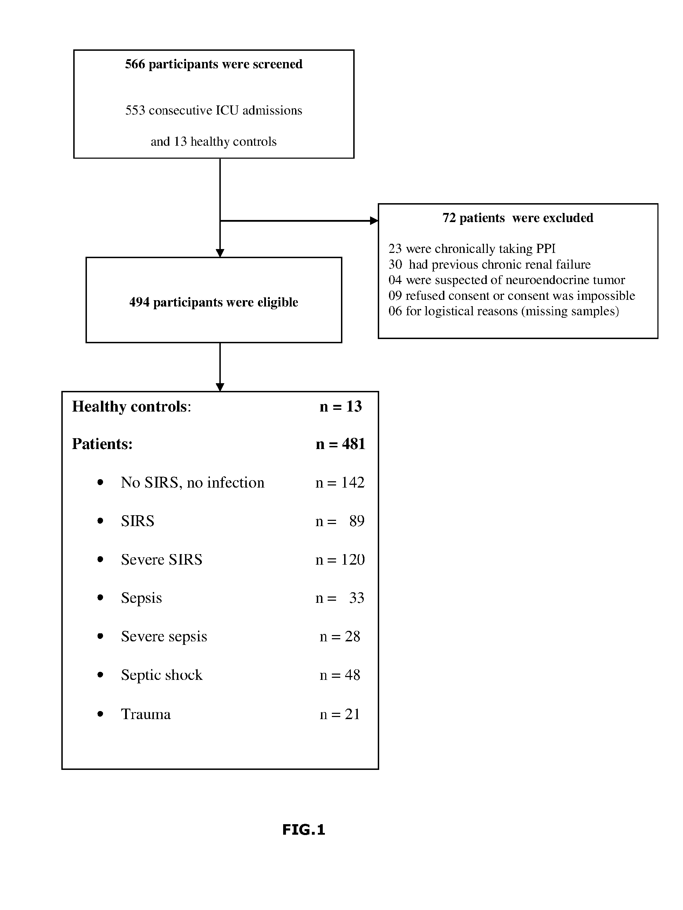

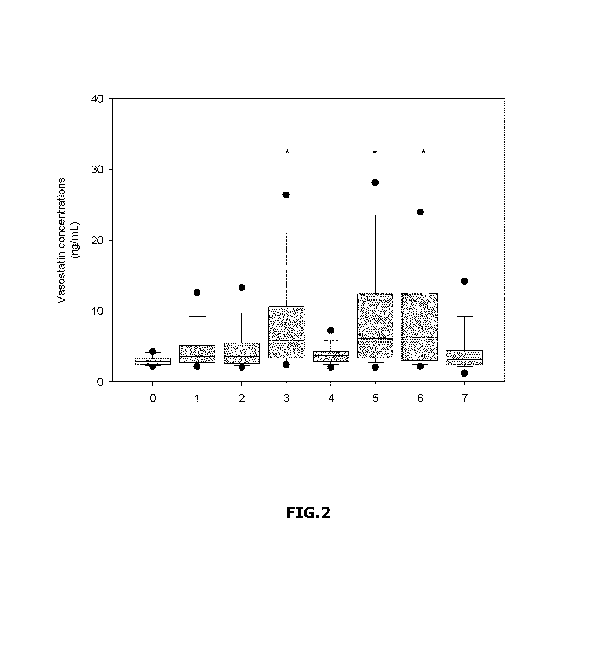

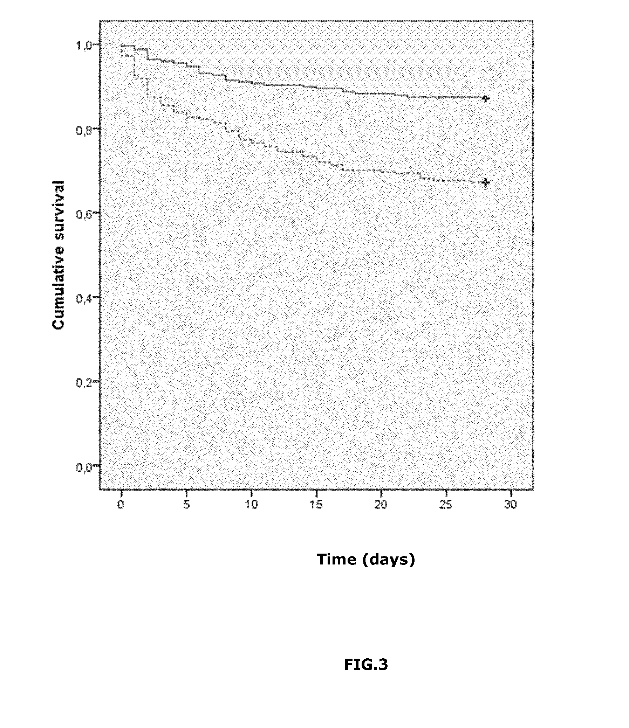

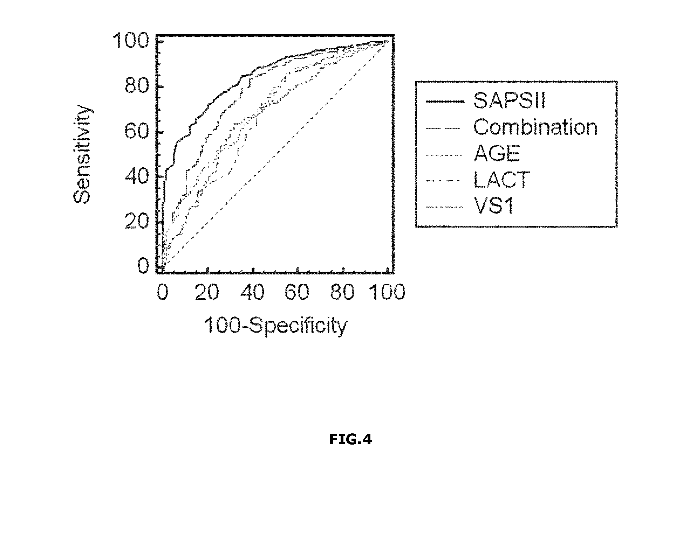

<u>Figure 1</u> **: Flow chart of the study**
ICU: Intensive Care Unit
PPI: Proton Pump Inhibitors
SIRS: Systemic Immuno-Inflammatory Syndrome
<u>Figure 2</u> : **Admission plasma VS-1 concentrations in the study population**
Comparison of median levels showed that the simultaneous presence of SIRS and circulatory failure criteria were required to have significantly different VS-1 concentrations in patients.
Data are presented as box plot representing the median and interquartile range [25; 75] of VS-1 with error bars representing $10^{th}$ and $90^{th}$ centiles.
Mann-Whitney test
*p< 0.01 for groups 3, 5 and 6 vs groups 0, 1, 2 and 4 ; and *p < 0.05 for group 3, 5 and 6 vs group 7.
0: healthy control (n=13); 1: no SIRS no infection (n=142); 2: SIRS (n=89); 3: severe SIRS (n=120); 4: sepsis (n=33); 5: severe sepsis (n=28); 6: septic shock (n=48); 7: trauma (n=21)
<u>Figure 3</u> **: Kaplan-Meier survival curves truncated at 28 Days for the median value of VS-1**
Patients with a VS-1 value lower than 3.97 [2.78; 7.41] ng/mL had an increased survival when compared to others (p<0.001, log-rank test).
<u>Figure 4</u> **: Receiver operating curves for VS-1, lactate, age and the SAPS II to predict mortality at Day 28.**
The AUC of the combination of admission VS-1 and lactate with age was significantly greater than the AUC of either VS-1, lactate or age taken alone (p<0.01). The gold standard in this setting, the SAPS II obtained 24h after admission, remained the best predictor (p<0.05).

## <u>EXAMPLES</u>

A pilot prospective observational study was conducted over six months in five intensive care units (ICU) of teaching hospitals.
**Inclusion Criteria** : Consecutive men and women over 18 years (Figure 1).
**Exclusion Criteria** : Patients were excluded if they were pregnant or had any conditions known to increase CgA independently of acute stress.

### Study protocol

Participants were categorised in 8 groups according to their clinical status: (0): healthy volunteers selected from the medical staff; (1) patients with neither systemic inflammatory response syndrome (SIRS) nor infection; (2) patients with SIRS; (3) patients with severe SIRS as defined in Intensive Care Med 2008;34:1654-61; (4-6) patients were considered septic only if an infectious agent could be identified in any biological sample from a focus of infection, and they were further classified into septic conditions according to standard definitions of sepsis, severe sepsis and septic shock as defined in Chest 1992;101:1481-1483; (7) trauma patients - these were previously healthy people involved in a trauma considered as the initial injury (plasma samples were collected within 6 h after the trauma).

A patient was considered to be in shock if mean arterial pressure was less than 70 mm Hg or the systolic blood pressure was less than 100 mm Hg despite the fact that an adequate amount of fluids (at least 1000 mL of crystalloids or 500 mL of colloids) had been administered (unless there was an elevation in the central venous pressure to > 12 mm Hg), and if there were signs of tissue hypoperfusion (e.g., altered mental state, mottled skin, urine output of <0.5mL per kg of bodyweight for 1h, or a serum lactate level>2mmol per L). In case of shock, patients were infused with norepinephrine to maintain mean arterial pressure over 65 mm Hg.

**Clinical and Biological Assessments**

Clinical status was assessed on admission. Main clinical data, and biological parameters were collected, and the SAPS II was generated 24 h after admission.

Standard biological data were necessary to assess the value of individual SAPS II scores. For this study, the following parameters were measured: C-reactive protein measured by immunoturbidimetry; creatinine measured by an enzymatic method (Siemens ADVIA, Paris, France); lactate measured using a lactate oxidase amperometric method (Roche Cobas b221, Meylan, France); and CgA measured using a kit CGA-ELISA (Cisbio Bioassays, Bagnols / Cèze, France).

**VS-1 (1-76) assay**

*Reagents*

96-well microplates Nunc Immobilized™, Streptavidin were used according to the manufacturer's recommendation. BSA free protease, goat anti-rabbit IgG-horseradish peroxidase conjugate (GAR-HRP), normal goat serum (NGS), N, N-Dimethylformamide anhydrous (DMF), aprotinin and Tween® 20 were from Sigma Aldrich (St. Louis, USA). Biotin-NHS was from Calbiochem (Darmstadt, Germany). Proclin was from Supelco (Pennsylvania, USA). EDTA and $H_2SO_4$ were obtained from Merck (Darmstadt, Germany). StabilZyme® HRP Conjugate Stabilizer was from Surmodics (Minnesota, USA). Chromogenic substrate TMB (3,3',5,5'-tetramethylbenzidine) was from KPL (Maryland, USA). The mouse monoclonal antibody (m5A8) raised against amino acids 53-57 of VS-1 (1-76) (Corti A et al. Eur J Biochem1997;248 (3):692-9), the rabbit polyclonal antibody raised against the C-terminal part (amino acids 70-76) of VS-1 and human recombinant VS-1 (1-76) from E.Coli were generously provided by Pr Corti (San Raffaele Institute, Milan). The rabbit polyclonal antibody was obtained by immunization with the VS-1 sequence 70-76 coupled to keyhole limpet hemocyanin (KLH).

*Antibodies and standard preparation*

The capture antibody (m5A8) was biotinylated prior to use. Briefly, after dialysis in buffer carbonate 100mM pH9, biotin-NHS dissolved in DMF was added to the antibody. After 30min of shaking, the free biotin was removed by dialysis in buffer phosphate buffer saline (PBS) 11mM pH 7.2. Aliquots of labeled antibody were frozen and stored at -20°C. The polyclonal antibody was diluted 5000 times in Phosphate 50mM +0.5%BSA +1% NGS, aliquoted and stored at -20°C.

Human recombinant VS-1 (1-76) was used as standard and diluted in dilution buffer (Phosphate 50mM +0.5%BSA +0.1% proclin+5mM EDTA+0.001% aprotinin), aliquoted and stored at -80°C.

The GAR-HRP was diluted in StabilZyme buffer and stored at 4°C.

Washing solution was $H_2O$+0.1%Tween.

*Assay procedure:*

The streptavidin coated microtiter plates were washed before use. 100μL of biotinylated capture antibody was added and incubated at room temperature (RT) for 1 hour with a 400rpm stirring. After washing, 100μL of calibrators or samples diluted in dilution buffer were added in duplicate and incubated 1 hour 30 minutes at RT, 400rpm. After another washing step, the captured VS-1 was recognized by the polyclonal antibody (100μL). After incubation (1 hour, RT, 400rpm), the unfixed reagents were eliminated by washing and 100μL of GAR-HRP was added for 30 minutes at RT, 400rpm. After a final washing step, 100μL of HRP substrate (TMB) was added. The chromogenic reaction was stopped with 100 μL $H_2SO_4$ 1N, and the absorbance of each sample was determined at 450 nm and 620nm (reference wavelength).

Blood samples were collected on admission into plasma-separator tubes (Becton Dickinson, France), immersed in ice and immediately shipped to the laboratory for processing. Plasma was separated by centrifugation at 1500 g for 10 min at room temperature and stored in 200 μL aliquots at -20°C until analysis. The VS-1 Elisa assay described above was used to assess VS-1 from these patients samples. Competition experiments showed that this ELISA did not cross-react with full-length recombinant human chromogranin A and with the vasostatin (1-78) fragment. The limit of detection was evaluated at 0.22 ng/mL and the intra and inter-assay coefficients of variation were lower than 4% and 7% respectively.

**Follow-up**

The length of stay and mortality were assessed at Day 28.

**Statistical methods**

Continuous variables were expressed as medians [interquartile range 25; 75]. The Mann-Whitney U test was used to compare continuous variables, and the chi-square test or Fisher's exact test was used to compare proportions. All statistical tests were 2-tailed, and the threshold of statistical significance was $P < 0.05$. Odds ratios (ORs) were calculated for variables with statistically significant differences between patients surviving or not at Day 28. Binary logistic regression was applied individually to each variable to obtain the OR in the univariate analysis. Statistically significant variables taken from the univariate analysis were introduced into a multivariate logistic regression model with forward stepwise logistic regression to identify the independent risk factors for survival. For each independent factor, receiver operating curves (ROC) were constructed, and the areas under the curve (AUC) assessed. For each variable the best cut-off value was chosen to construct a predictive model of outcome assessment. This score was then tested in the whole cohort to establish a threshold of best sensitivity/specificity of the predictive score. AUC were compared using the Z statistic with correction for the correlation introduced by studying the same sample. Probabilities of survival were estimated by the Kaplan-Meier method and were compared with a log-rank test.

**RESULTS**

The data of 481 patients and 13 healthy staff form the basis of this study. Among them, 13 patients demonstrating a severe SIRS and circulatory failure with (n=5) or without (n=8) infection were randomly chosen for serial twice-a-day samplings for VS-1 over the first 3 days following admission.

**Epidemiologic and clinical data.**

Main epidemiologic, clinical data are summarized in Table 2 according to survival. In the Table the following abbreviations are used: Vs-1: vasostatin-1; CGA: chromogranin A; SAPS II: Simplified Acute Physiology Score. Data are median and percentiles [25; 75] for continuous variables, and numbers (or percentages).
Mann-Whitney test: p indicated in the table.

**Table 2: Main characteristics of patients on admission according to mortality at day 28.**

| Parameters | Alive (n = 368) | Dead (n = 113) | P value |
|---|---|---|---|
| **Age** | 59.3 [43.8-72.5] | 74.8 [59.7-82.4] | < 0.001 |
| **SAPS II** | 38 [25-52] | 66 [53-82] | < 0.001 |
| **Reasons for referral** | | | |
| • Severe infection (n=109): | | | < 0.05 |
| ○ Sepsis | 30 | 3 | |
| ○ Severe sepsis | 24 | 4 | |
| ○ Septic shock | 32 | 16 | |
| • No SIRS No infection (n= 142) | 125 | 17 | |
| • SIRS (n=89) | 75 | 14 | < 0.001 |
| • Severe SIRS (n=120) | 64 | 56 | |
| • Trauma (n=21) | 18 | 3 | |
| **Biological data** | | | |

(continued)

| Parameters | Alive (n = 368) | Dead (n = 113) | P value |
|---|---|---|---|
| VS-1 (ng/mL) | 3.7 [2.7; 6.12] | 5.75 [3.65; 11.2] | < 0.001 |
| C-reactive protein (mg/L) | 45.0 [9.4; 154.0] | 46.4 [13.2; 169.5] | 0.730 |
| Lactate (mmol/L) | 1.8 [1.1; 3.2] | 3.9 [1.5; 6.6] | < 0.001 |
| White blood cell counts (Giga/L) | 12.9 [8.9; 17.3] | 14.8 [7.9; 18.7] | 0.308 |
| Creatinine ($\mu$mol/L) | 79.8 [55.0; 137.2] | 133.0 [86.3; 228.5] | < 0.001 |
| CGA (ng/mL) | 83.0 [42.5; 202.0] | 121.5 [60.0; 275.0] | < 0.05 |

The overall mortality was 23.5%, 23% in the serial measurement-patients. Survivors were significantly younger, and with a lower SAPS II score. Of note, 22.7 % of all admission were due to severe infection (sepsis n=33; severe sepsis n=28; septic shock n=48) with positive bacterial, viral or fungal samples and the simultaneous presence of a sure focus of infection. 43.4 % of the patients were admitted with fever and SIRS criteria but without any focus of infection and without any positive sample for infection (SIRS n= 89; severe SIRS n=120). Many, if not all these patients, were already infused antibiotics before admission, which means that admission samples may have been negative due to these treatments. The most common causes of referral for non-infectious presenting medical problems were as follows:

- In the no SIRS no infection group: neurological causes (ischaemic or haemorrhagic stroke and status epilepticus 28%); acute respiratory failure 27% (including alveolar hypoventilation in COPD and acute cardiogenic pulmonay oedema); self-poisoning with or without alcohol 25%; metabolic causes 9.5% (including acute renal failure); and miscellaneous 9.5%.
- In the SIRS group: acute respiratory failure 28%; emergency abdominal surgery 19%; out-of-hospital cardiac arrest 17%; self-poisoning with aspiration 11%; metabolic causes 10%; and miscellaneous 15% (including mild liver failure and/or pa ncreatitis).
- In the severe SIRS group: out-of-hospital cardiac arrest 22%; emergency surgery 20%; acute respiratory failure 17% (including mostly ARDS of non-infectious cause); neurologic causes 11%; and miscellaneous 30% (including severe acute liver failure and pancreatitis).

**Distribution of plasma VS-1 concentrations**

*Single admission values*

The median plasma VS-1 levels for healthy staff were 2.85 [2.44; 3.34] ng/mL, whereas the values for critically ill patients were 4.06 [2.78; 7.61] ng/mL (p<0.001). As indicated (Figure 2), only severe SIRS patients (5.77 [3.31; 10.63] ng/mL), severe sepsis (6.07 [3.18; 12.56] ng/mL), and septic shock patients (6.21 [2.96; 13.10] ng/mL) displayed significantly (p<0.01) higher plasma VS-1 concentrations when compared to no SIRS no infection patients (3.56 [2.63; 5.12] ng/mL), and sepsis patients (3.6 [2.84; 4.30] ng/mL). Trauma patients had no significant increase in VS-1 when compared to healthy staff (3.13 [2.38; 4.75], p=0.101); they were nevertheless significantly lower when compared to those noted in severe SIRS, severe sepsis and septic shock patients (p<0.05). Finally, there was a significant increase in all patients demonstrating circulatory failure when compared to those without circulatory failure (5.93 [3.30; 11.06] ng/mL vs 3.58 [2.59; 5.05] ng/mL, p<0.001), and also in non survivors versus survivors (5.75 [3.65; 11.20] ng/mL vs 3.70 [2.67; 6.12] ng/mL, p<0.001), but there was no significant difference whether infection was present or not (4.41 [2.93; 9.59] ng/mL vs 3.88 [2.72; 6.96] ng/mL, p=0.547).

**Mortality and VS-1**

Admission VS-1 levels significantly increased in non survivors when compared to survivors (Table 1). In addition (Figure 3), survival rates were significantly worse in our cohort in those patients with an admission VS-1 level above the median value of 3.97 ng/mL (p<0.001, log-rank test). Of note is the fact that a similar result was obtained for patients with a lactate value above the median value (1.85 mmol/L, p<0.001, not shown).

Using a Cox regression model, age and concentrations of VS-I and lactate were independent predictors of mortality on Day 28 after admission (p<0.01). The relative risks (95% CI) of death were respectively 1.035 (1.017-1.053) for VS-1, 1.045 (1.031-1.059) for age, and 1.114 (1.081-1.148) for lactate.

Using ROC curves (Figure 4), we found that taken independently, admission values of VS-1, lactate and age were of equivalent value for the prediction of mortality (AUC respectively 0.705 for age; 0.669 for VS-1; 0.668 for lactate), and significantly weaker than the gold standard SAPS II (AUC =0.845, p<0.05) (Figure 4). When combining age, admission lactate and VS-1 levels, using the best threshold for each independent factor of mortality prediction taken separately, we could construct a predictive score for the combination which turned out to be significantly better than each factor taken alone ($p < 0.01$). Moreover, results obtained from this combination were available only 5h after admission whereas the SAPS II could only be achieved at Day +1.

**Claims**

1. An *in vitro* method for determining if a patient is at risk of increased mortality, said method comprising the step of:

    (a) measuring the level of vasostatin-1 in a biological sample obtained from said patient.

2. The method according to claims 1, further comprising the steps of:

    (b) comparing the measured level of vasostatin-1 in the sample with a predetermined value measured in a control population of healthy subjects or critically ill survivors; and
    (c) correlating levels of vasostatin-1 with the patient's mortality risk.

3. The method according to claim 2, wherein step (b) further comprises comparing the age of the patient with a predetermined cut-off age and wherein step (c) consists in correlating age and level of vasostatin-1 with the patient's mortality risk.

4. An *in vitro* method for determining if a patient is at risk of increased mortality, said method comprising the steps of:

    (a) measuring the level of vasostatin-1 in a biological sample obtained from said patient;
    (b) measuring the level of lactate in a biological sample obtained from said patient.

5. The method according to claims 4, further comprising the steps of:

    (c) comparing the measured level of vasostatin-1 and lactate in the samples with predetermined values of each marker measured in control populations of healthy subjects or critically ill survivors; and
    (d) correlating levels of vasostatin-1 and of lactate with the patient's mortality risk.

6. The method according to claim 5, wherein step (c) further comprises comparing the age of the patient with a predetermined cut-off age and wherein step (d) consists in correlating age, levels of vasostatin-1 and levels of lactate with the patient's mortality risk.

7. A method of monitoring the decrease or increase of a patient mortality risk, comprising practicing the method according to any one of claims 1 to 6 with a first and a second or further sample from the same patient, said first and second or further samples being obtained at different times, correlating the level(s) of vasostatin-1, optionally levels of lactate and/or age with the patient's mortality risk for each of the first or second or further sample, and then comparing the patient's mortality risk determined with each sample.

8. The method according to any one of claims 1 to 7, wherein said step of measuring the level of vasostatin-1 comprises the steps of:

    (i) contacting said biological sample with a compound capable of binding vasostatin-1;
    (ii) detecting the level of vasostatin-1 present in the sample by observing the level of binding between said compound and vasostatin-1.

9. The method according to claim 8, wherein said compound capable of binding vasostatin-1 is an antibody raised against vasostatin-1 or synthetic peptides derived therefrom.

10. The method according to claim 9 wherein said antibody's epitope is located within amino acids 66-76 of VS-1.

11. The method according to any one of claims 1 to 7, wherein said step of measuring the level of vasostatin-1 is performed using a sandwich immunoassay that includes a first antibody whose epitope is located within amino acids 66-76 of VS-1 and a second antibody whose epitope is located within amino-acids 1-66 of VS-1.

**12.** The method according to any one of claims 1 to 11, wherein said patient is a critically ill patient.

**13.** The method according to any one of claims 1 to 12, wherein said patient is affected with severe SIRS, severe sepsis or severe shock.

**14.** The method according to any one of claims 1 to 13, wherein the biological sample is a whole blood sample, a serum sample, or a plasma sample.

**15.** A vasostatin-1 sandwich immunoassay kit comprising a first antibody whose epitope is located within amino acids 66-76 of vasostatin-1, and a second antibody whose epitope is located within amino acids 1-66 of vasostatin-1.

**16.** The vasostatin-1 sandwich immunoassay kit according to claim 15, wherein said first antibody's epitope is located within amino acids 70-76 of vasostatin-1.

| | |
|---|---|
| **566 participants were screened** | |
| 553 consecutive ICU admissions | |
| and 13 healthy controls | |

**72 patients were excluded**

23 were chronically taking PPI
30 had previous chronic renal failure
04 were suspected of neuroendocrine tumor
09 refused consent or consent was impossible
06 for logistical reasons (missing samples)

**494 participants were eligible**

| **Healthy controls:** | **n = 13** |
|---|---|
| **Patients:** | **n = 481** |
| • No SIRS, no infection | n = 142 |
| • SIRS | n = 89 |
| • Severe SIRS | n = 120 |
| • Sepsis | n = 33 |
| • Severe sepsis | n = 28 |
| • Septic shock | n = 48 |
| • Trauma | n = 21 |

**FIG.1**

**FIG.2**

**Time (days)**

**FIG.3**

**FIG.4**

European Patent Office
Europäisches Patentamt
European Patent Office
Office européen des brevets

## EUROPEAN SEARCH REPORT

Application Number

EP 12 30 5056

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 792 638 A1 (INST NAT SANTE RECH MED [FR]) 27 October 2000 (2000-10-27) * claims 1-3,8-11,13; sequences 1-4 * | 15,16 | INV. G01N33/74 |
| Y | DI COMITE GABRIELE ET AL: "Chromogranin A: a novel factor acting at the cross road between the neuroendocrine and the cardiovascular systems", JOURNAL OF HYPERTENSION, LIPPINCOTT WILLIAMS & WILKINS, LTD, US / UK, vol. 29, no. 3, 1 March 2011 (2011-03-01), pages 409-414, XP008151786, ISSN: 0263-6352, DOI: 10.1097/HJH.0B013E328341A429 | 1-14 | |
| A | * page 410; figure 1 * * page 409, column 2, line 6 - paragraph 2 * | 15,16 | |
| X | SILVESTRO ROATTA ET AL: "The chromogranin A- derived N-terminal peptide vasostatin-I:effects on cardiovascular variables in the rabbit", REGULATORY PEPTIDES, ELSEVIER SCIENCE BV, NL, vol. 168, no. 1, 18 February 2011 (2011-02-18), pages 10-20, XP028206106, ISSN: 0167-0115, DOI: 10.1016/J.REGPEP.2011.02.015 [retrieved on 2011-02-27] * page 12, column 1, paragraph 3 * | 15,16 | TECHNICAL FIELDS SEARCHED (IPC) G01N |
| Y | WO 2008/145701 A1 (INST NAT SANTE RECH MED [FR]; SCHNEIDER FRANCIS [FR]; METZ MARIE-HELEN) 4 December 2008 (2008-12-04) * abstract * * claim 1; table 1 * * page 25, paragraph 4 * | 1-14 | |

-/--

The present search report has been drawn up for all claims

| Place of search | Date of completion of the search | Examiner |
|---|---|---|
| The Hague | 21 May 2012 | Gundlach, Björn |

EPO FORM 1503 03.82 (P04C01)

Europäisches
Patentamt

European
Patent Office

Office européen
des brevets

# EUROPEAN SEARCH REPORT

Application Number

EP 12 30 5056

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VINCENT J-L: "Lactate levels in critically ill patients", ACTA ANAESTHESIOLOGICA SCANDINAVICA, WILEY-BLACKWELL MUNKSGAARD, DK, vol. 39, no. SUPPL. 107, 1 September 1995 (1995-09-01), pages 261-266, XP008151814, ISSN: 0001-5172, DOI: 10.1111/J.1399-6576.1995.TB04368.X [retrieved on 2008-12-30] * abstract * * page 265, column 2, paragraph 1 * ----- | 4 | |
| Y | DE BACKER D: "Lactic acidosis", MINERVA ANESTESIOLOGICA, EDIZIONI MINERVA MEDICA, IT, vol. 69, no. 4, 1 April 2003 (2003-04-01), pages 281-284, XP008151779, ISSN: 0375-9393 * abstract * ----- | 4 | |
| Y | FUNK GEORG CHRISTIAN ET AL: "Acid-base disturbances in critically ill patients with cirrhosis", LIVER INTERNATIONAL, BLACKWELL MUNKSGAARD, OXFORD, GB , vol. 27, no. 7 1 September 2007 (2007-09-01), pages 901-909, XP008151807, ISSN: 1478-3223, DOI: DOI:10.1111/J.1478-3231.2007.01510.X Retrieved from the Internet: URL:http://www3.interscience.wiley.com/cgi -bin/issn?DESCRIPTOR=PRINTISSN& VALUE=1478-3223 [retrieved on 2007-06-05] * abstract * ----- | 4 | TECHNICAL FIELDS SEARCHED (IPC) |

The present search report has been drawn up for all claims

| Place of search | Date of completion of the search | Examiner |
|---|---|---|
| The Hague | 21 May 2012 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 30 5056

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2012

| Patent document cited in search report | | Publication date | Patent family member(s) | | Publication date |
|---|---|---|---|---|---|
| FR 2792638 | A1 | 27-10-2000 | FR | 2792638 A1 | 27-10-2000 |
| | | | WO | 0064939 A2 | 02-11-2000 |
| WO 2008145701 | A1 | 04-12-2008 | EP | 2153233 A1 | 17-02-2010 |
| | | | JP | 2010528307 A | 19-08-2010 |
| | | | US | 2010196921 A1 | 05-08-2010 |
| | | | WO | 2008145701 A1 | 04-12-2008 |

EPO FORM P0459

For more details about this annex : see Official Journal of the European Patent Office, No. 12/82

## REFERENCES CITED IN THE DESCRIPTION

**Patent documents cited in the description**

- WO 2008145701 A

- US 4946778 A

**Non-patent literature cited in the description**

- **BRUN-BUISSON.** *Intensive Care Med.,* 2000, vol. 26 (1), 64-74
- **HARBARTH et al.** *Am J Respir Crit Care Med.,* 2001, vol. 164, 396-402
- **SCHETZ et al.** *Int J Artif Organs,* 2005, vol. 28 (2), 1197-210
- **KNAUS et al.** *Crit Care Med.,* 1981, vol. 9, 591-597
- **LE GALL et al.** *JAMA,* 1993, vol. 270, 2957-2963
- **LEMESHOW et al.** *Crit Care Med.,* 1988, vol. 16, 470-477
- **FERREIRA et al.** *JAMA,* 2001, vol. 286, 1754-1758
- **TIMSIT et al.** *Crit Care Med,* 2002, vol. 30, 2003-1 3
- **ROATTA et al.** *Regul Pept.,* 07 June 2011, vol. 168 (1-3), 10-20

- **PENCINA et al.** Evaluating the added predictive ability of a new marker: from area under the ROC curve to reclassification and beyond. *Stat Med.,* 2008, vol. 27, 157-172
- **KOHLER et al.** *Nature,* 1975, vol. 256 (5517), 495-7
- **COTE et al.** *Proc Natl Acad Sci U S A.,* 1983, vol. 80 (7), 2026-30
- **COLE et al.** Monoclonal Antibodies and Cancer Therapy. Alan Liss, Inc, 1985, 77-96
- **TUERK et al.** *Science,* 1990, vol. 249, 505-510
- **JAYASENA.** *Clin Chem.,* 1999, vol. 45 (9), 1628-50
- **COLAS et al.** *Nature,* 1996, vol. 380, 548-50
- *Intensive Care Med,* 2008, vol. 34, 1654-61
- *Chest,* 1992, vol. 101, 1481-1483
- **CORTI A et al.** *Eur J Biochem,* 1997, vol. 248 (3), 692-9